# EUROPEAN PATENT APPLICATION

(11) **EP 4 732 795 A2**
(43) Date of publication of application: **29.04.2026**
(21) Application number: 25211505.0
(22) Date of filing: 27.10.2025
(51) Int. Cl.: A61B 17/72

(54) **SCREW TRAJECTORIES IN ANKLE ARTHRODESIS NAIL**

(30) Priority: 28.10.2024 US 202463712775 P
(71) Applicant: Zimmer, Inc., Warsaw, IN 46580 (US)
(72) Inventor: VAN DYKE, William Scott, Warsaw, 46580 (US); LIPORACE, Frank A, Englewood Cliffs, 07632 (US)
(74) Representative: Taor, Simon Edward William

(57) **Abstract**

An ankle arthrodesis implant can include an elongate shaft sized and shaped to be implanted in an intramedullary canal of a tibia of a patient; a first angled opening through the shaft, the first angled opening defining a first axis that extends through the tibia and into a talus bone of the patient across a tibio-talar joint when the elongate shaft is implanted; a first elongate slot through the shaft, the first elongate slot defining a second axis that extends into the patient's talus when the elongate shaft is implanted, the first elongate slot being configured to allow a first fixation member to translate axially relative to the shaft within the first slot from a first position to a second different position to compress a tibio-talar joint, wherein the second axis is perpendicular to a sagittal plane of the ankle; a second elongate slot through the shaft, the second elongate slot defining a third axis that extends into a patient's calcaneus when the elongate shaft is implanted, the second elongate slot being configured to allow a second fixation member to translate axially relative to the shaft within the second slot from a first position to a second different position to compress a subtalar joint; a second angled opening through the shaft, the second angled opening defining a fourth axis that extends through the calcaneus and into a talus bone of the patient across the subtalar joint when the elongate shaft is implanted; and a third angled opening through the shaft, the third angled opening defining a fifth axis that extends into a calcaneus bone of the patient when the elongate shaft is implanted.

## Description

### TECHNICAL FIELD

The present disclosure relates to surgical implant systems. Specifically, the present disclosure relates to an ankle arthrodesis system.

### BACKGROUND

Intramedullary implants are used in various contexts, including to repair fractures or to achieve joint fusion (*i.e*., arthrodesis). In the case of arthrodesis, it is frequently necessary to apply compression across one or more joints to bring the adjoining bones of the joint into apposition, and then fix the adjoining bones so that fusion can occur across the joint site. An intramedullary implant can be used to achieve fixation of adjoining bones of a joint after compression and secure the bones in position to allow fusion to occur.

Ankle arthrodesis is a procedure where either the subtalar joint or both the tibiotalar (ankle) joint and subtalar joint are fused. These procedures are limb salvage procedures either due to disease or a failed total ankle arthroplasty. To obtain a good fusion, the joint surfaces need prepared and reduced with good bone on bone contact through compression. One means of locking the compression across the joint surfaces is by using screws which span the joint lines.

### OVERVIEW

To further illustrate apparatuses and methods disclosed herein, a non-limiting list of examples is provided here:
Example 1 is an ankle arthrodesis implant including an elongate shaft sized and shaped to be implanted in an intramedullary canal of a tibia of a patient; a first angled opening through the shaft, the first angled opening defining a first axis that extends through the tibia and into a talus bone of the patient across a tibio-talar joint when the elongate shaft is implanted; a first elongate slot through the shaft, the first elongate slot defining a second axis that extends into the patient's talus when the elongate shaft is implanted, the first elongate slot being configured to allow a first fixation member to translate axially relative to the shaft within the first slot from a first position to a second different position to compress a tibio-talar joint, wherein the second axis is perpendicular to a sagittal plane of the ankle; a second elongate slot through the shaft, the second elongate slot defining a third axis that extends into a patient's calcaneus when the elongate shaft is implanted, the second elongate slot being configured to allow a second fixation member to translate axially relative to the shaft within the second slot from a first position to a second different position to compress a subtalar joint; a second angled opening through the shaft, the second angled opening defining a fourth axis that extends through the calcaneus and into a talus bone of the patient across the subtalar joint when the elongate shaft is implanted; and a third angled opening through the shaft, the third angled opening defining a fifth axis that extends into a calcaneus bone of the patient when the elongate shaft is implanted.
Example 2 is the implant of Example 1, wherein optionally the second axis is parallel to a transverse plane of an ankle.
Example 3 is the implant of any of Examples 1-2, wherein optionally second axis is parallel to a coronal plane of the ankle.
Example 4 is the implant of any of Example 1-3, wherein optionally the elongate shaft includes an internal bore, wherein the internal bore is threaded.
Example 5 is the implant of any of Examples 1-4, further including first, second, third, fourth, and fifth fixation members each defining a diameter, wherein the first and second elongate slots each has a length that is anywhere between about 125-500% of the diameter of the second and third fixation members.
Example 6 is the implant of any of Examples 1-5, wherein optionally the first axis defines a 45-degree descending angle relative to a longitudinal axis of the elongate shaft, a 28-degree post-lateral to antemedial angle, and the first angled opening is positioned 62.5 mm from a distal end of the elongate shaft.
Example 7 is the implant of any of Examples 1-6, wherein optionally the first elongate slot includes a 7 mm compression slot positioned to allow a fixation member to translate from 40 mm to 47 mm from the distal end of the elongate shaft.
Example 8 is the implant of any of Examples 1-7, wherein optionally the second elongate slot includes a 4 mm compression slot positioned to allow a fixation member to translate from 16.5 mm to 20.5 mm from the distal end of the elongate shaft.
Example 9 is the implant of any of Examples 1-8, wherein optionally the fourth axis opening defines a 25-degree ascending angle relative to the longitudinal axis of the elongate shaft, a 13-degree post-lateral to antemedial angle, and the second angled opening is positioned 28 mm from a distal end of the elongate shaft.
Example 10 is the implant of any of Examples 1-9, wherein optionally the fifth axis defines a 5-degree ascending angle relative to the longitudinal axis of the elongate shaft, a 5-degree post-lateral to antemedial angle, and the third angled opening is positioned 10.5 mm from a distal end of the elongate shaft.
Example 11 is the implant of any of Examples 1-10, wherein optionally the implant is configured to cover at least 80% of a population of ankles.
Example 12 is an ankle arthrodesis implant including an elongate shaft sized and shaped to be implanted in an intramedullary canal of a tibia of a patient; a first angled opening through the shaft, the first angled opening defining a first axis that extends through the tibia and into a talus bone of the patient across a tibio-talar joint when the elongate shaft is implanted, the first angled opening positioned 62.5 mm from a distal end of the elongate shaft; a first elongate slot through the shaft, the first elongate slot defining a second axis that extends into the patient's talus when the elongate shaft is implanted, the first elongate slot being configured to allow a first fixation member to translate axially relative to the shaft within the first slot from a first position to a second different position to compress a tibio-talar joint, wherein the first elongate slot includes a 7 mm compression slot positioned to allow the first fixation member to translate from 40 mm to 47 mm from the distal end of the elongate shaft; a second elongate slot through the shaft, the second elongate slot defining a third axis that extends into a patient's calcaneus when the elongate shaft is implanted, the second elongate slot being configured to allow a second fixation member to translate axially relative to the shaft within the second slot from a first position to a second different position to compress a subtalar joint, wherein the second elongate slot includes a 4 mm compression slot positioned to allow the second fixation member to translate from 16.5 mm to 20.5 mm from the distal end of the elongate shaft; a second angled opening through the shaft, the second angled opening defining a fourth axis that extends through the calcaneus and into a talus bone of the patient across the subtalar joint when the elongate shaft is implanted, wherein the second angled opening is positioned 28 mm from a distal end of the elongate shaft; and a third angled opening through the shaft, the third angled opening defining a fifth axis that extends into a calcaneus bone of the patient when the elongate shaft is implanted, wherein the third angled opening is positioned 10.5 mm from a distal end of the elongate shaft.
Example 13 is the implant of Example 12, wherein optionally the second axis is perpendicular to a sagittal plane of the ankle and parallel to a transverse plane of the ankle.
Example 14 is the implant of any of Examples 12-13, wherein optionally the first axis defines a 45-degree descending angle relative to a longitudinal axis of the shaft and a 28-degree post-lateral to antemedial angle, and the fourth axis defines a 25-degree ascending angle relative to the longitudinal axis of the shaft and a 13-degree post-lateral to antemedial angle, and the fifth axis defines a 5-degree ascending angle relative to the longitudinal axis of the shaft and a 5-degree post-lateral to antemedial angle.
Example 15 is a method of implanting an intramedullary implant including implanting an intramedullary implant into an intramedullary canal of a tibia, the intramedullary implant having a shaft and an internal bore; fastening a proximal end of the intramedullary implant to the tibia; inserting a first fixation member into a first elongate slot extending through the shaft and into a talus and translating the first fixation member axially relative to the shaft within the first elongate slot from a first position to a second different position to compress a tibio-talar joint, wherein the first elongate slot is perpendicular to a sagittal plane of the ankle; after inserting the first fixation member, inserting a second fixation member into a second elongate slot extending through the shaft and into a calcaneus and translating the second fixation member axially relative to the shaft within the second elongate slot from a first position to a second different position to compress a subtalar joint; after inserting the second fixation member, inserting a third fixation member into a second angled opening through the shaft, the second angled opening defining a second angled opening axis that extends through the calcaneus and into a talus bone of the patient across the subtalar joint when the intramedullary implant is implanted; inserting a fourth fixation member into a third angled opening through the shaft, the third angled opening defining a third angled opening axis that extends into a calcaneus bone of the patient when the intramedullary implant is implanted; and inserting a fifth fixation member into a first angled opening through the shaft, the first angled opening defining a first angled opening axis that extends through the tibia and into a talus bone of the patient across a tibio-talar joint when the intramedullary implant is implanted.
Example 16 is the method of Example 15, wherein optionally the first angled opening axis defines a 45-degree descending angle relative to a longitudinal axis of the shaft, a 28-degree post-lateral to antemedial angle, and is positioned 62.5 mm from a distal end of the elongate shaft.
Example 17 is the method of any of Examples 15-16, wherein optionally the first elongate slot includes a 7 mm compression slot positioned to allow the first fixation member to translate from 40 mm to 47 mm from a distal end of the elongate shaft.
Example 18 is the method of any of Examples 15-17, wherein optionally wherein the second elongate slot includes a 4 mm compression slot positioned to allow the second fixation member to translate from 16.5 mm to 20.5 mm from a distal end of the elongate shaft.
Example 19 is the method of any of Examples 15-18, wherein optionally the second angled opening axis defines a 25-degree ascending angle relative to the longitudinal axis of the shaft, a 13-degree post-lateral to antemedial angle, and is positioned 28 mm from a distal end of the elongate shaft.
Example 20 is the method of any of Examples 15-19, wherein optionally the third angled opening axis defines a 5-degree ascending angle relative to the longitudinal axis of the shaft, a 5-degree post-lateral to antemedial angle, and is positioned 10.5 mm from a distal end of the elongate shaft.
In Example 21, the apparatuses or methods of any one or any combination of Examples 1-20 can optionally be configured such that all elements or combinations of elements or options recited are available to use or select from.

These and other examples and features of the present apparatuses and methods will be set forth in part in the following Detailed Description. This Overview is intended to provide non-limiting examples of the present subject matter - it is not intended to provide an exclusive or exhaustive explanation. The Detailed Description below is included to provide further information about the present apparatus, systems, and methods.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 shows a medial to lateral view of an intramedullary implant, in accordance with one embodiment.
FIG. 2 shows a posterior to anterior view of the intramedullary implant of FIG. 1, in accordance with one embodiment.
FIG. 3 shows a cross-section view of the intramedullary implant, in accordance with one embodiment.
FIG. 4 shows a lateral view of an implanted intramedullary implant, in accordance with one embodiment.
FIG. 5 shows a posterior view of the implanted intramedullary implant, in accordance with one embodiment.
FIG. 6 shows a top view of the implanted intramedullary implant, in accordance with one embodiment.
FIG. 7 shows a method for implanting an ankle arthrodesis implant, in accordance with one embodiment.

### DETAILED DESCRIPTION

As used herein, the following directional definitions apply. Anterior and posterior mean nearer the front or nearer the rear of the body, respectively, proximal and distal mean nearer to or further from the root of a structure, respectively, and medial and lateral mean nearer the sagittal plane or further from the sagittal plane, respectively. The sagittal plane is an imaginary vertical plane through the middle of the body that divides the body into right and left halves.

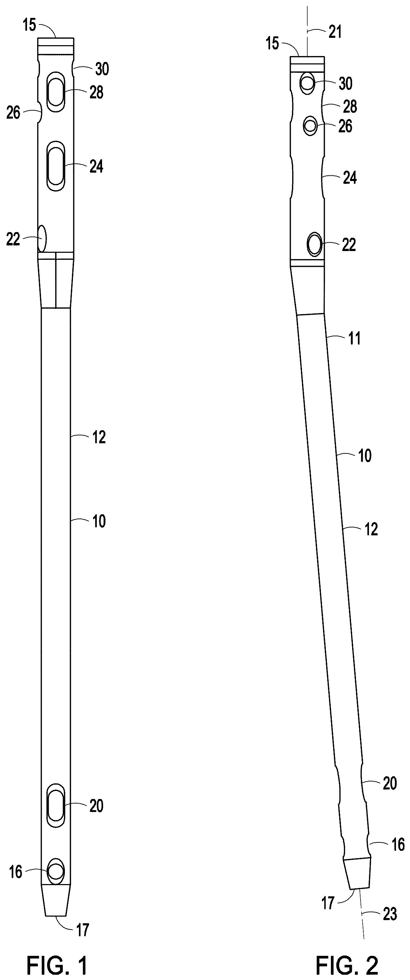

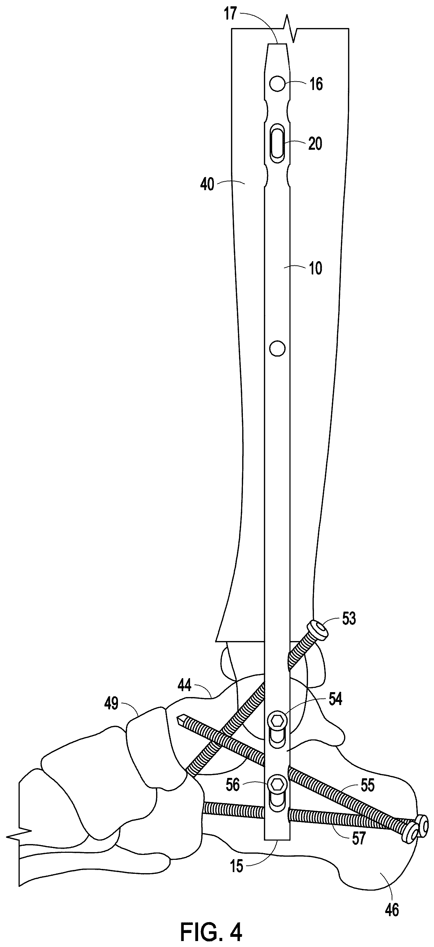

FIGs 1, 2, and 3 illustrate an intramedullary implant 10 in the form of an intramedullary nail (*e.g*., an IM ankle nail). FIGs 4, 5, and 6 illustrate the implant 10 as implanted into a tibia.

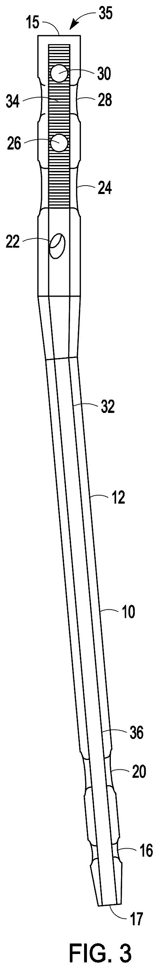

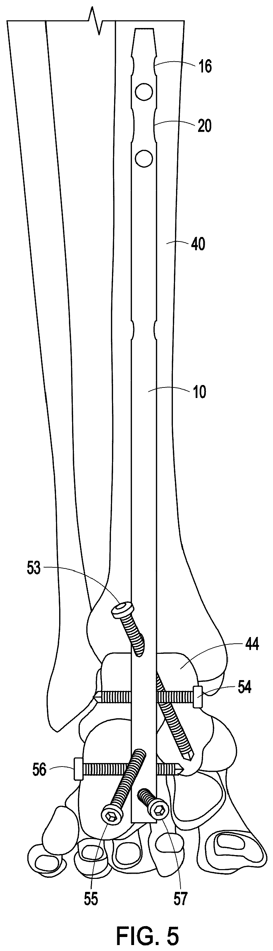

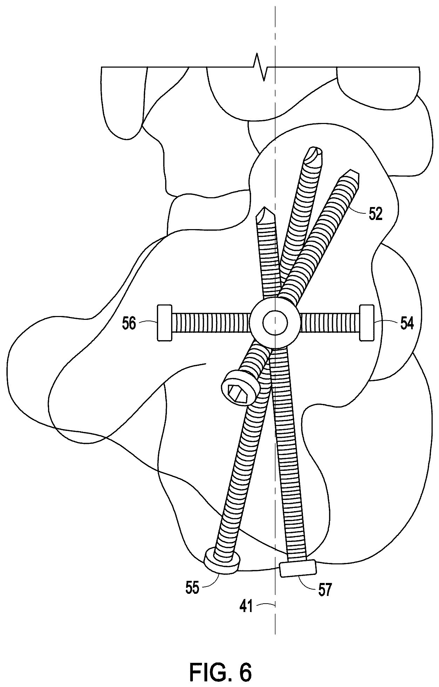

Specifically, FIG. 1 shows a medial to lateral view of an intramedullary implant, in accordance with one embodiment, FIG. 2 shows a posterior to anterior view of the intramedullary implant of FIG. 1, FIG. 3 shows a cross-section view of the intramedullary implant, FIG. 4 shows a lateral view of an implanted intramedullary implant, FIG. 5 shows a posterior view of the implanted intramedullary implant, and FIG. 6 shows a top view of the implanted intramedullary implant, in accordance with one embodiment.

As will be discussed, the present intramedullary implant 10 has unmatched screw trajectories in the distal end of the nail. The present system has both a lateral-to-medial slot in the talus to perform internal ankle joint compression and a lateral-to-medial slot in the calcaneus to perform internal subtalar joint compression. Presently, no available nail systems allow for internal joint compression for the subtalar joint. Instead, available systems usually rely on external compression on the skin of the hindfoot.

Moreover, the present system provides a posterior-to-anterior screw in the calcaneus with a slight deviation posteromedial to antero-lateral and a slight ascending angle. Finally, there are two screws that span the two joints that are intending to be fused. First, there is a calc-talar screw which is ascending and posterolateral to antero-medial in trajectory. Second, there is a tibiotalar screw which is descending and also posterolateral to antero-medial in trajectory. This screw is a similar concept to a "homerun" screw, where a screw that starts in the tibial metaphysis ends up in the talar neck.

In this example, the implant 10 can have a shaft 12 extending from a proximal end 15 to a distal end 17 with a bend 11 between a proximal section of shaft 12 and a distal section of shaft 12. Bend 11 can be anywhere between about two degrees to about fifteen degrees (2-15°), in another example anywhere between about five degrees to about ten degrees (5-10°), or in yet another example about five degrees (5°). Thus, the proximal section of shaft 12 can define a first axis 21 and the distal section of shaft 12 can define a second axis 23, and the axes 21,23 can intersect to form any of the aforementioned angles. Implant 10 can also include a set of distal openings 16, 20 for receiving bone screws (not shown) to attach the distal end of the implant 10 to a tibia, as known in the art. Any of openings 16, 20 can be threaded or unthreaded, in any combination. Openings 16, 20 can also be arranged substantially perpendicular to the second axis 23, in an example, or any of openings 16, 20 can be an elongate compression slot.

Implant 10 can further include a first angled opening 22 through the shaft 12 at the proximal section of shaft 12. In an example, opening 22 can be angled through shaft 12 proximally by between about thirty-five degrees to about fifty degrees (35-50°), or alternatively about forty five degrees (45°) proximally relative to axis 21. Opening 22 can also be angled approximately anywhere between about twenty degrees to about thirty degrees (20-30°), in an example about twenty eight degrees (28°), medially relative to a sagittal plane 41 extending through the patient's ankle, as illustrated in FIG. 6. Further, the opening 22 can be positioned 62.5 mm from the distal end 15 of the elongate shaft 12.

As shown in FIGs 4-6, this can create a situation in which a bone screw 53 can extend along a first axis defined by the angled opening 22 and can be inserted downwardly through opening 22 and into a patient's tibia 40 and talus 44. As noted, the first angled opening 22 is about 62.5 mm from the distal end 15 of the implant 10 (as measured at a midpoint of the shaft 12). The angle of the opening 22 allows the screw 53 to extend into tibia 40 and talus 44 when inserted through opening 22. In another example, screw 53 can have a length to extend into the patient's navicular 49 - *i.e.,* through tibia 40, talus 44, and into navicular 49. In an example, it can be said that an imaginary central axis of opening 22 can extend and/or project through tibia 40, talus 44, and navicular 49 (*e.g*., so that a certain size screw can extend into the foregoing bones, depending upon its length).

Thus, screw 53 includes a posterolateral to anteromedial screw in the descending direction that captures the anterior third of the talus (head and neck area) and starts in the superior aspect of the tibial pilon. In some examples, depending on the size of the screw 53, there is also the potential of capturing the anterior portion of the navicular-talar joint. This screw 53 also counters the plantar flexion force of the foot. Many surgeons use adjunctive anterior plates in a TTC joint fusion for additional fixation. Screw 53 can prevent the surgeon from needing this supplemental fixation.

Implant 10 can also include a first elongate slot 24 that is elongated along the first axis 21. Slot 24 can have a width that is approximately the same as a bone screw 54 and a length that is greater than the width, in an example a length that is anywhere between about one-hundred and twenty five percent to four hundred percent (125-400%) of the diameter of the bone screw 54. The elongate length of slot 24 can allow bone screw 54 to move longitudinally within slot 24 by anywhere between about two millimeters to about ten millimeters (2-10 mm), in an example about seven millimeters (7 mm). In one example, the elongate slot 24 can be dimensioned to allow the screw 54 to translate about from 40 mm from the distal end 15 to 47 mm from the distal end 15. Such movement of the screw 54 can act to compress a patient's talus 44 against the patient's tibia 40. Thus, the first elongate slot 24 can include a 7 mm compression slot positioned to allow the screw 54 to translate from 40 mm to 47 mm from the distal end 15 of the elongate shaft 12.

Further, as shown in FIG. 6, a central axis of slot 24, as exemplified by the position of the screw 54, can be angled medially relative to sagittal plane 41 by about ninety degrees (90°). Thus the central axis of the slot 24 is perpendicular to the sagittal plane 41. This creates a situation in which the screw 54 inserted through the slot 24 extends along the same medial angle as slot 24.

Thus when implanted, as shown in FIGs 4-6, the first elongate slot 24 defines an axis that extends into the patient's talus 44 when the elongate shaft 12 is implanted. The first elongate slot 24 is configured to allow a fixation member such as the screw 54 to translate relative to the shaft 12 within the first slot from a first position to a second different position to compress a tibio-talar joint. In this example, the axis defined by the elongate slot 24 is perpendicular to the sagittal plane 41 of the ankle, and is parallel to a transverse plane of an ankle and is parallel to a coronal plane of the ankle.

The implant 10 can also have a second angled opening 26 extending through the shaft 12, which is arranged distally of slot 24. Opening 26 can be angled distally relative to the axis 21 by anywhere between about twenty degrees to about thirty five degrees (20-35°), in an example about twenty five degrees (25°). Further, opening 26 can be angled medially relative to sagittal plane 41, as shown in FIG. 6, by anywhere between about ten degrees to about twenty five degrees (10-25°), in an example about thirteen degrees (13°). As shown in FIGs 4-6, this can create a situation in which a screw 55 inserted through opening 26 ascends upwards along the axis of the angled opening 26 and can extend through a patient's calcaneus 46 and into the patient's talus 44. In an example, the opening 26 can be anywhere between about twenty-five millimeters to about thirty millimeters (25-30) from the distal end 15, in a specific example about twenty-eight millimeters (28 mm) from the distal end 15 (as measured to a mid-point of the shaft 12).

Thus, the second angled opening 26 defines an axis that extends through the calcaneus and into a talus bone of the patient across the subtalar joint when the elongate shaft 12 is implanted. The opening 26 allows for a posterolateral to anteromedial screw 55 to be implanted in the ascending direction which spans the subtalar joint.

The implant can further include a second elongate slot 28 disposed through the shaft 12 of the implant 10. Slot 28 can have a width that is approximately the same as a bone screw 56 and a length that is greater than the width, in an example a length that is anywhere between about one-hundred and twenty five percent to four hundred percent (125-400%) of the diameter of bone screw 56. The elongate length of slot 28 can allow bone screw 56 to move longitudinally within slot 28 by anywhere between about two millimeters to about ten millimeters (2-10 mm), in an example about four millimeters (4 mm). Such movement of screw 56, can act to compress a patient's calcaneus 46 against the patient's talus 44. Further, as shown in FIG. 6, a central axis of slot 28 can extend roughly perpendicular to sagittal plane 41 so that the screw 56 can extend through calcaneus 46 in a medial-to-lateral direction. In an example, the elongate opening 28 can extend anywhere between about fifteen millimeters to about twenty five millimeters (15-25 mm) from the distal end 15 of the implant 10, in a specific example the elongate opening 28 can be dimensioned to allow the bone screw 56 to translate from about sixteen and a half to about twenty and a half millimeters (16.5 mm-20.5 mm) from the distal end 15.

Thus, the second elongate slot 28 defines an axis that extends into a patient's calcaneus 46 when the elongate shaft 12 is implanted. The second elongate slot 28 is configured to allow a second fixation member such as the screw 56 to translate relative to the shaft 12 within the second slot 28 from a first position to a second different position to compress the subtalar joint (the joint between the calcaneus 46 and the talus 44). In this example, the second elongate slot 28 includes a 4 mm compression slot positioned to allow the screw 56 to translate from 16.5 mm to 20.5 mm from the distal end 15 of the elongate shaft 15. The axis defined by the elongate slot 28, as illustrated by the screw 56 in FIGs 4-6 is perpendicular to the sagittal plane 41 of the ankle, is parallel to the transverse plane of the ankle, and is parallel to a coronal plane of the ankle.

Implant 10 can further include a third angled opening 30. Opening 30 can be angled distally relative to axis 21 by anywhere between about two degrees to about fifteen degrees (2-15°), in an example about five degrees (5°). In addition, opening 30 can be angled laterally relative to sagittal plane 41 by anywhere between about two degrees to about fifteen degrees (2-15°), in an example about five degrees (5°). As shown in FIGs 4-6, this can create a situation in which a screw 57 inserted into the opening 30 and extending along an axis defined by the opening 30 can extend upwards and laterally by the above angle amounts in the patient's calcaneus 46. In an example, the opening 30 can be anywhere between about ten millimeters to about 12 millimeters (10-12 mm) from the distal end 15, in a specific example 10 and a half millimeters (10.5 mm) from the distal end 15 (as measured to a mid-point of the shaft 12). In another example, screw 57 can be of a sufficient length to extend through calcaneus 46 and into a patient's cuboid across the calcaneus-cuboid joint.

Thus, the angled opening 30 defines an axis that extends into a calcaneus 46 bone of the patient when the elongate shaft 12 is implanted. In this example, the axis of the opening 30 defines a 5-degree ascending angle relative to the axis 21, a 5-degree post-lateral to antemedial angle, and is positioned 10.5 mm from a distal end of the elongate shaft.

In a study of a library of six common adult ankles, the implant 10, including the screw openings discussed above, covered at least 80% of a population of ankles in the library. Specifically, an implant with the first opening 22 having a 45-degree descending angle and a 28-degree posterolateral to anteromedial angle, positioned 62.5 mm from the distal end of the nail, the first elongate slot 24 having 7 mm of compression 40-47 mm from the distal end 15, the second elongate slot 28 having 4 mm of compression 16.5 - 20.5 mm from the distal end 15 of the implant 10, the second angled opening 26 having a 25-degree ascending angle and a 13-degree posterolateral to anteromedial angle, positioned 28 mm from the distal end 15 of the implant 10, and the third angled opening 30 having a 5-degree ascending angle and a 5-degree posteromedial to anterolateral angle, and positioned 10.5 mm from the distal end 15 of the implant 10 hit the desired ankle bone landmarks in five out of the six ankles in the library.

As shown in FIG. 3, the implant 10 can also be hollow, such that a bore 32 extends through the shaft 12. Bore 32 can define a first distal bore part 35 and a second proximal bore part 36. Further, a portion or all of the distal proximal bore part 35 can be threaded 34. In an example, threading 34 can extend from the distal end 15 end of the distal bore part 35 and encompass part or all of the walls defining distal bore part 35 along the extent of the elongate slot 24. Likewise, threading 34 can extend along part or all of the walls defining distal bore part 35 along the extent of the elongate slot 28. Subsequently, such threading 34 can be used with CoreLock^{™} Technology, developed by Biomet, Inc.

Briefly, CoreLock^{™} Technology is exemplified in U.S. Pat. No. 9,308,031 ("the '031 Patent"), the disclosure of which is hereby incorporated by reference herein. By CoreLock^{™} Technology, Applicant is referring to any of the telescopic clamps, securing devices, locking devices, and/or compression devices disclosed in the '031 Patent, for example securing devices 200 *(see e.g.,* FIG. 16 of the '031 Patent), 700 (*see e.g.,* FIGs 25-26), and 900 (*see e.g.,* FIGs 34-35) and locking device 1030 (*see e.g.,* FIGs 44-44A) and compression device 1024 (*see e.g*., FIGs 45-45A). It is to be understood that the aforementioned securing, locking, and compression devices of the '031 Patent can be used with implant 10 in ways that would be appreciated by a person of skill in the art, in varying combinations. Thus, implant 10 can have characteristics that make it suitable for use with such securing, locking, and compression devices. For example, a portion of the bore 32 *- e.g.,* distal bore part 35 - can be keyed as is described with reference to, amongst other areas, FIGs 47A-47B of the '031 Patent. As set forth in the '031 Patent, for example with reference to locking device 1030 and compression device 1024, a portion of longitudinal bore 1006 of IM implant 1002 can be keyed as at 1020, 1022 so as to key with second component 1078 of locking device 1030 and/or second component 1076 of compression device 1024. This creates a situation in which first components 1050, 1046 of the locking and compression devices 1030, 1024 can be rotated relative to second components 1078, 1076 and, by virtue of the threaded interaction between first components 1050, 1046 and the IM longitudinal bore 1006, locking and compression devices 1030, 1024 can be moved longitudinally relative to the IM implant. Such movement can cause screws associated with the respective securing, compression, or locking device to be secured, compressed in a particular direction within a slot of the IM implant, or locked, respectively. As described in more detail below, Applicant contemplates that such mechanisms can be used herein to lock and/or cause translation of any of screws 53-57 within openings 22, 26, 30 and slots 24, 28. Certain examples from the '031 Patent might be used for simplicity's sake, but it is to be understood that any of the securing, locking, and/or compression devices of the '031 Patent can be used with implant 10 and the instrumentation described herein.

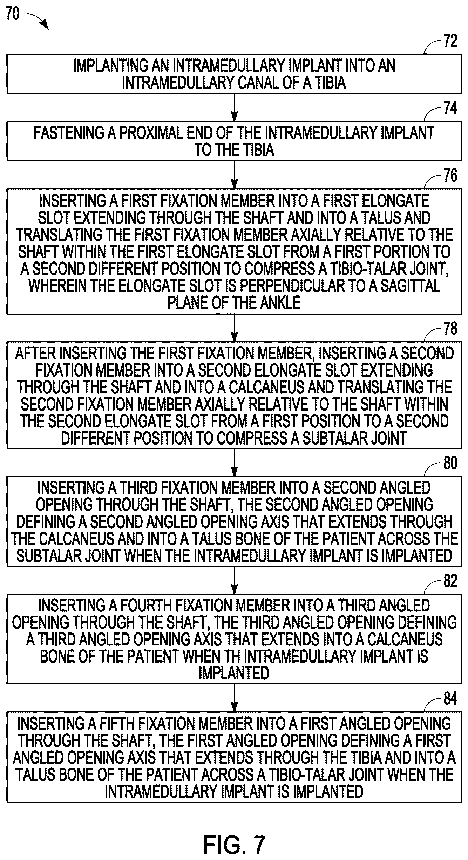

FIG. 7 shows a method (70) for implanting an ankle arthrodesis implant, in accordance with one embodiment.

In general, the method will be described with reference to the implant 10 being an ankle arthrodesis nail. First, a surgeon can make an incision in a patient's foot, for example the plantar aspect of a patient's foot, and prep the various bones to receive the implant 10. This can involve drilling a pilot hole and/or reaming out a bore of a sufficient size to receive implant 10. The reamed bore can extend through the patient's calcaneus 46, talus 44, and into tibia 40 a sufficient distance for implant 10 to reside in the bore.

Referring to FIGs 1-6, the method (70) can include implanting an intramedullary implant (72) into an intramedullary canal of a tibia, the intramedullary implant having a shaft and an internal bore. The method further includes fastening a proximal end of the intramedullary implant to the tibia (74), for example, at the proximal holes 16, 20 of the implant 10. The method further includes inserting a first fixation member 54 into a first elongate slot (76) extending through the shaft and into a talus and translating the first fixation member axially relative to the shaft within the first elongate slot from a first position to a second different position to compress a tibio-talar joint, wherein the first elongate slot is perpendicular to a sagittal plane of the ankle. After inserting the first fixation member, the method includes inserting a second fixation member 56 into a second elongate slot (78) extending through the shaft and into a calcaneus and translating the second fixation member axially relative to the shaft within the second elongate slot from a first position to a second different position to compress a subtalar joint. After inserting the second fixation member, the method (70) includes inserting a third fixation member 55 into a second angled opening (82) through the shaft, the second angled opening 26 defining a second angled opening axis that extends through the calcaneus and into a talus bone of the patient across the subtalar joint when the intramedullary implant is implanted, inserting a fourth fixation member 57 into a third angled opening (86) through the shaft, the third angled opening 30 defining a third angled opening axis that extends into a calcaneus bone of the patient when the intramedullary implant is implanted, and inserting a fifth fixation member 53 into a first angled opening (80) through the shaft, the first angled opening 22 defining a first angled opening axis that extends through the tibia and into a talus bone of the patient across a tibio-talar joint when the intramedullary implant is implanted.

As discussed above, in various embodiment of the method (70), the first angled opening axis can define a 45-degree descending angle relative to the axis of the shaft, a 28-degree post-lateral to antemedial angle, and can be positioned 62.5 mm from a distal end of the elongate shaft. The first elongate slot can include a 7 mm compression slot positioned to allow the first fixation member to translate from 40 mm to 47 mm from a distal end of the elongate shaft. The second elongate slot can include a 4 mm compression slot positioned to allow the second fixation member to translate from 16.5 mm to 20.5 mm from a distal end of the elongate shaft.

In some examples, the second angled opening axis can define a 25-degree ascending angle, a 13-degree post-lateral to antemedial angle, and can be positioned 28 mm from a distal end of the elongate shaft. The third angled opening axis can define a 5-degree ascending angle, a 5-degree post-lateral to antemedial angle, and be positioned 10.5 mm from a distal end of the elongate shaft.

With the above construct, the tibio-talar joint and the subtalar joint can be compressed internally in two stages, and various screws can be used along with implant 10 to fix the compression and the ankle joint (*e.g*., for ankle fusion to occur). In some examples, suitable bone graft material or other augments can be used to assist with the fusion.

In summary, present implant 10 provides for unmatched screw trajectories in the distal end of the nail. The present system has both a lateral-to-medial slot in the talus to perform internal ankle joint compression and a lateral-to-medial slot in the calcaneus to perform internal subtalar joint compression. Presently, no available nail systems allow for internal joint compression for the subtalar joint. Instead, available systems usually rely on external compression on the skin of the hindfoot.

Moreover, the present system provides a posterior-to-anterior screw in the calcaneus with a slight deviation posteromedial to antero-lateral and a slight ascending angle. Finally, there are two screws that span the two joints that are intending to be fused. First, there is a calc-talar screw which is ascending and posterolateral to antero-medial in trajectory. Second, there a tibiotalar screw which is descending and also posterolateral to antero-medial in trajectory. This screw is a similar concept to a "homerun" screw, where a screw that starts in the tibial metaphysis ends up in the talar neck.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls. In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

## Claims

1. An ankle arthrodesis implant comprising:
an elongate shaft sized and shaped to be implanted in an intramedullary canal of a tibia of a patient;
a first angled opening through the shaft, the first angled opening defining a first axis that extends through the tibia and into a talus bone of the patient across a tibio-talar joint when the elongate shaft is implanted;
a first elongate slot through the shaft, the first elongate slot defining a second axis that extends into the patient's talus when the elongate shaft is implanted, the first elongate slot being configured to allow a first fixation member to translate axially relative to the shaft within the first slot from a first position to a second different position to compress a tibio-talar joint, wherein the second axis is perpendicular to a sagittal plane of the ankle;
a second elongate slot through the shaft, the second elongate slot defining a third axis that extends into a patient's calcaneus when the elongate shaft is implanted, the second elongate slot being configured to allow a second fixation member to translate axially relative to the shaft within the second slot from a first position to a second different position to compress a subtalar joint;
a second angled opening through the shaft, the second angled opening defining a fourth axis that extends through the calcaneus and into a talus bone of the patient across the subtalar joint when the elongate shaft is implanted; and
a third angled opening through the shaft, the third angled opening defining a fifth axis that extends into a calcaneus bone of the patient when the elongate shaft is implanted.

2. The implant of claim 1, wherein the second axis is parallel to a transverse plane of an ankle.

3. The implant of claim 2, wherein the second axis is parallel to a coronal plane of the ankle.

4. The implant of claim 1, wherein the elongate shaft includes an internal bore, wherein the internal bore is threaded.

5. The implant of claim 1, further including first, second, third, fourth, and fifth fixation members each defining a diameter, wherein the first and second elongate slots each has a length that is anywhere between about 125-500% of the diameter of the second and third fixation members.

6. The implant of claim 1, wherein the first axis defines a 45-degree descending angle relative to a longitudinal axis of the elongate shaft, a 28-degree post-lateral to antemedial angle, and the first angled opening is positioned 62.5 mm from a distal end of the elongate shaft.

7. The implant of claim 6, wherein the first elongate slot includes a 7 mm compression slot positioned to allow a first fixation member to translate from 40 mm to 47 mm from the distal end of the elongate shaft.

8. The implant of claim 7, wherein the second elongate slot includes a 4 mm compression slot positioned to allow a second fixation member to translate from 16.5 mm to 20.5 mm from the distal end of the elongate shaft.

9. The implant of claim 8, wherein the fourth axis opening defines a 25-degree ascending angle relative to the longitudinal axis of the elongate shaft, a 13-degree post-lateral to antemedial angle, and the second angled opening is positioned 28 mm from a distal end of the elongate shaft.

10. The implant of claim 9, wherein the fifth axis defines a 5-degree ascending angle relative to the longitudinal axis of the elongate shaft, a 5-degree post-lateral to antemedial angle, and the third angled opening is positioned 10.5 mm from a distal end of the elongate shaft.

11. The implant of claim 10, wherein the implant is configured to cover at least 80% of a population of ankles

12. An ankle arthrodesis implant comprising:
an elongate shaft sized and shaped to be implanted in an intramedullary canal of a tibia of a patient;
a first angled opening through the shaft, the first angled opening defining a first axis that extends through the tibia and into a talus bone of the patient across a tibio-talar joint when the elongate shaft is implanted, the first angled opening positioned 62.5 mm from a distal end of the elongate shaft;
a first elongate slot through the shaft, the first elongate slot defining a second axis that extends into the patient's talus when the elongate shaft is implanted, the first elongate slot being configured to allow a first fixation member to translate axially relative to the shaft within the first slot from a first position to a second different position to compress a tibio-talar joint, wherein the first elongate slot includes a 7 mm compression slot positioned to allow a first fixation member to translate from 40 mm to 47 mm from the distal end of the elongate shaft;
a second elongate slot through the shaft, the second elongate slot defining a third axis that extends into a patient's calcaneus when the elongate shaft is implanted, the second elongate slot being configured to allow a second fixation member to translate axially relative to the shaft within the second slot from a first position to a second different position to compress a subtalar joint, wherein the second elongate slot includes a 4 mm compression slot positioned to allow a second fixation member to translate from 16.5 mm to 20.5 mm from the distal end of the elongate shaft;
a second angled opening through the shaft, the second angled opening defining a fourth axis that extends through the calcaneus and into a talus bone of the patient across the subtalar joint when the elongate shaft is implanted, wherein the second angled opening is positioned 28 mm from a distal end of the elongate shaft; and
a third angled opening through the shaft, the third angled opening defining a fifth axis that extends into a calcaneus bone of the patient when the elongate shaft is implanted, wherein the third angled opening is positioned 10.5 mm from a distal end of the elongate shaft.

13. The implant of claim 12, wherein the second axis is perpendicular to a sagittal plane of the ankle and parallel to a transverse plane of the ankle.

14. The implant of claim 12, wherein the first axis defines a 45-degree descending angle relative to a longitudinal axis of the shaft and a 28-degree post-lateral to antemedial angle, and the fourth axis defines a 25-degree ascending angle relative to the longitudinal axis of the shaft and a 13-degree post-lateral to antemedial angle, and the fifth axis defines a 5-degree ascending angle relative to the longitudinal axis of the shaft and a 5-degree post-lateral to antemedial angle.
